# EUROPÄISCHE PATENTSCHRIFT

(11) **EP 2 604 171 B1**
(45) Veröffentlichungstag und Bekanntmachung des Hinweises auf die Patenterteilung: **04.06.2014**
(21) Anmeldenummer: 12008093.2
(22) Anmeldetag: 04.12.2012
(51) Int. Cl.: A61B 1/00, A61B 17/00

(54) **Handgriff für ein medizinisches Instrument**
Handle for a medical instrument
Poignée pour un instrument médical

(30) Priorität: 17.12.2011 DE 102011121498
(43) Veröffentlichungstag der Anmeldung: 19.06.2013
(73) Patentinhaber: Karl Storz GmbH & Co. KG, 78532 Tuttlingen (DE)
(72) Erfinder: Hermle,Rainer, 78559 Gosheim (DE); Merz, Robin, 78120 Furtwangen (DE)
(74) Vertreter: Hofmeister, Frank

(56) Entgegenhaltungen:
- EP-A2- 2 382 940
- DE-A1- 19 805 532

## Beschreibung

Die Erfindung betrifft einen Handgriff für ein medizinisches Instrument mit einem Griffgehäuse und einem in das Griffgehäuse einsetzbaren Anschlussadapter für Schlauch- und/oder Rohranschlüsse, wobei der Anschlussadapter über mindestens einen Anschlussstutzen fluiddicht in eine entsprechende Stutzenaufnahme des Griffgehäuses einsetzbar ist und am mindestens einen Anschlussstutzen eine umlaufende Ringnut zur Aufnahme eines Dichtungselements angeordnet ist, wobei in Axialrichtung des mindestens einen Anschlussstutzens am Anschlussstutzen mindestens zwei hintereinander angeordnete umlaufende Ringnuten ausgebildet sind, wobei der Anschlussstutzen im Bereich der mindestens zwei Ringnuten einen gleichbleibenden Außendurchmesser aufweist, und wobei zumindest in der vom freien Ende des Anschlussstutzens aus betrachtet ersten Ringnut ein Dichtungselement, insbesondere ein O-Ring, angeordnet ist

Saug- und Spülhandgriffe für medizinische Instrumente sind aus der Praxis in verschiedenen Ausführungsformen bekannt. Die ausgehend von einer externen Spülflüssigkeitsquelle über den Handgriff hin zum distalen Ende des medizinischen Instruments geführte Spülflüssigkeit dient während der Operation unter anderem der Spülung des Operationssitus sowie dazu, eine Endoskopoptik zu reinigen, um dem Operateur einen stets ungetrübten Blick auf das Operationsgebiet zu gewährleisten. Über einen Saugkanal werden die Spülflüssigkeit und auch Blut aus dem Operationsgebiet abgesaugt, wobei der Saugkanal über den Handgriff an eine externe Saugvorrichtung angeschlossen ist.

Zur Verbindung des Handgriffs mit der externen Spülflüssigkeitsquelle und der externen Saugvorrichtung dienen Schlauchleitungen, die proximal handgriffseitig an einem im Griffgehäuse angeordneten Anschlussadapter festgelegt werden. Über einen distalseitig am Anschlussadapter angeordneten Anschlussstutzen wird der Anschlussadapter fluiddicht in eine Stutzenaufnahme des Griffgehäuses eingesetzt.

Da der Anschlussadapter nur schlecht oder gar nicht vollständig gereinigt werden kann, ist der Anschlussadapter in der Regel als auswechselbares Einwegteil ausgebildet, um diesen leicht einbauen und wieder ausbauen zu können. Bei den aus der Praxis bekannten Handgriffen erfolgt die fluiddichte Abdichtung im Bereich des Anschlussstutzens des Anschlussadapters über ein vorzugsweise als O-Ring ausgebildetes Dichtungselement, das in einer Ringnut des Anschlussstutzens angeordnet ist.

Ein gattungsgemäßer Stand der Technik ist beispielsweise aus der EP 2 382 940 A2 bekannt. Diese bekannte Elektromotoranordnung weist ein Motorgehäuse mit einem Kupplungszapfen auf, über den das Motorgehäuse in einem Handstück des Dentalinstruments festlegbar ist.

Der Kupplungszapfen seinerseits weist in Axialrichtung des Kupplungszapfens mehrere hintereinander angeordnete umlaufende Ringnuten auf, in denen jeweils ein O-Ring als Dichtungselement angeordnet ist. Somit zeigt dieses Dokument den Stand der Technik, der der vorliegenden Erfindung zugrunde lag.

Das Problem bei der Verwendung der O-Ringdichtung ist, dass der O-Ring beim Einsetzen des Anschlussstutzens in die Stutzenaufnahme des Griffgehäuses eine Gleitreibung erzeugt, die aufgrund der sehr kleinen Baumaße der medizinischen Instrumente das Einsetzen erschwert oder sogar unmöglich macht.

Davon ausgehend liegt der Erfindung die **Aufgabe** zugrunde, einen Handgriff der eingangs genannten Art zu schaffen, dessen Anschlussadapter bei leichter Montier- und Demontierbarkeit einen fluiddichten Anschluss gewährleistet.

Die **Lösung** dieser Aufgabenstellung ist erfindungsgemäß dadurch gekennzeichnet, dass das mindestens eine Dichtungselement beim Einsetzen des Anschlussstutzens des Anschlussadapters in die Stutzenaufnahme des Griffgehäuses durch Gleitreibung aus seiner Lage in der jeweiligen Ringnut in eine in Axialrichtung des Anschlussstutzens nach proximal direkt anschließende Ringnut überführbar ist.

Durch die Anordnung der mindestens zwei hintereinander angeordneten umlaufenden Ringnuten und des mindestens einen in der distalseitig vordersten Ringnut angeordneten Dichtungselements wird beim Einschieben des Anschlussstutzens in die Stutzenaufnahme die auftretende Gleitreibung zwischen der Oberfläche des Dichtungselements und der Innenseite der Stutzenaufnahme dazu verwendet, das Dichtungselement von der ersten in die zweite Ringnut zu verschieben bzw. zu rollen. Auf diese Weise wird der Widerstand beim Einsetzen des Anschlussadapters in das Griffgehäuse verringert und das Dichtungselement trotzdem in einer genau definierten fluiddicht abdichtenden Position gehalten.

Um erfindungsgemäß eine fluiddichte Abdichtung des Anschlussadapters zu gewährleisten, ist das mindestens eine Dichtungselement beim Einsetzen des Anschlussstutzens des Anschlussadapters in die Stutzenaufnahme des Griffgehäuses aus seiner Lage in der jeweiligen Ringnut in eine in Axialrichtung des Anschlussstutzens nach proximal direkt anschließende Ringnut überführbar.

Da es sich einerseits bei den als Einwegteilen hergestellten Anschlussadaptern in der Regel um Spritzgussteile handelt, die von der Produktion her höhere Toleranzen als Fräs- oder Drehteile aufweisen und andererseits die vorzugsweise als O-Ringe ausgebildete Dichtungselemente ebenfalls nicht einheitlich ausgebildet sind, ist das Zusammenspiel von Nut und O-Ring besonders undefiniert. Erst durch die erfindungsgemäße Ausbildung der zweiten Ringnut in die das Dichtungselement bei der Montage überführt wird, ist es möglich, die geforderte Fluiddichtigkeit zum Handgriff zu gewährleisten.

Um zu bewirken, dass das vorzugsweise als O-Ring ausgebildete Dichtungselement beim Übergang von der ersten Ringnut in die zweite Ringnut nicht bloß axial verschoben wird, sondern von der einen Ringnut in die andere Ringnut rollt, wird gemäß einer bevorzugten Ausgestaltung der Erfindung vorgeschlagen, dass die hintereinander angeordneten Ringnuten durch jeweils ein Stoppelement voneinander getrennt sind, das vorteilhafterweise als radiale Erhebung ausgebildet ist. Da das Stoppelement in Relation zum Dichtungselement nur sehr klein, also in axialer Richtung schmal und radial flach ausgebildet ist, ist die Kraft, die benötigt wird, um das Dichtungselement über das Stoppelement zu schieben bzw. zu rollen, relativ gering. Durch die Bewegung des Einschiebens des Anschlussstutzens in die Stutzenaufnahme wird das Dichtungselement gegen das Stoppelement gedrückt und dreht sich mindestens um seinen halben Umfang. Um wieder in den Normalzustand zu gelangen, "springt" das Dichtungselement quasi in die zweite Ringnut, so dass das Dichtungselement bei vollständig eingesetzem Anschlussadapter nach etwa einer vollen Umdrehung fest und gut abdichtend in der zweiten Ringnut sitzt.

Vorteilhafterweise sind die hintereinander angeordneten Ringnuten parallel zueinander angeordnet, jedoch ist auch eine schräg zueinander verlaufende Anordnung der Ringnuten möglich.

Mit einer praktischen Ausführungsform der Erfindung wird vorgeschlagen, dass die Ringnuten gleich breit und in radialer Richtung gleich tief ausgebildet sind, um eine definierte Lage des Dichtungselements in der jeweiligen Ringnut sicherzustellen.

Gemäß einer praktischen Ausführungsform der Erfindung wird vorgeschlagen, dass am Anschlussstutzen zwei in Axialrichtung parallel zueinander angeordnete umlaufende Ringnuten ausgebildet sind und in der distalseitig vorderen Ringnut ein als O-Ring ausgebildetes Dichtungselement angeordnet ist.

Schließlich wird mit der Erfindung vorgeschlagen, dass der Anschlussadapter auswechselbar im Griffgehäuse festlegbar ist. Die auswechselbare Ausgestaltung des Anschlussadapters ermöglicht die Ausbildung des Anschlussadapters als Einwegbauteil, das nach jeder Behandlung demontiert und weggeworfen wird, da dessen sterile Reinigung kaum oder aber nur mit einem sehr großen Aufwand möglich wäre.

Um eine fluiddichte Abdichtung des Anschlussadapters zu gewährleisten, wird mit der Erfindung vorgeschlagen, dass das mindestens eine Dichtungselement beim Einsetzen des Anschlussstutzens des Anschlussadapters in die Stutzenaufnahme des Griffgehäuses aus seiner Lage in der jeweiligen Ringnut in eine in Axialrichtung des Anschlussstutzens nach proximal direkt anschließende Ringnut überführbar ist.

Weiterhin wird mit der Erfindung vorgeschlagen, dass das mindestens eine Dichtungselement beim Herausziehen des Anschlussstutzens des Anschlussadapters aus der Stutzenaufnahme des Griffgehäuses wieder zurück in die ursprüngliche Ringnut überführbar ist. Durch das reversible Zurückführen des mindestens einen Dichtungselement in seine ursprüngliche Ringnut wird sichergestellt, dass die Fluiddichtigkeit auch beim erneuten Einsetzen des Anschlussadapters wieder gewährleistet ist.

Weitere Merkmale und Vorteile der Erfindung ergeben sich anhand der zugehörigen Zeichnungen, in denen drei Ausführungsbeispiel eines erfindungsgemäßen Handgriffs nur beispielhaft dargestellt ist, ohne die Erfindung auf diese Ausführungsbeispiele zu beschränken. In den Zeichnungen zeigt:
- Fig. 1: eine Seitenansicht eines erfindungsgemäßen Handgriffs;
- Fig. 2: eine Draufsicht auf einen Anschlussadapter gemäß einer ersten erfindungsgemäßen Ausführungsform;
- Fig. 3: eine vergrößerte Ansicht des Details III gemäß Fig. 2;
- Fig. 4: einen Schnitt entlang der Linie IV-IV gemäß Fig. 1;
- Fig. 5: eine vergrößerte Ansicht des Details V gemäß Fig. 4;
- Fig. 6: eine Darstellung gemäß Fig. 3, jedoch eine zweite erfindungsgemäße Ausführungsform darstellend und
- Fig. 7: eine Darstellung gemäß Fig. 3, jedoch eine dritte erfindungsgemäße Ausführungsform darstellend.

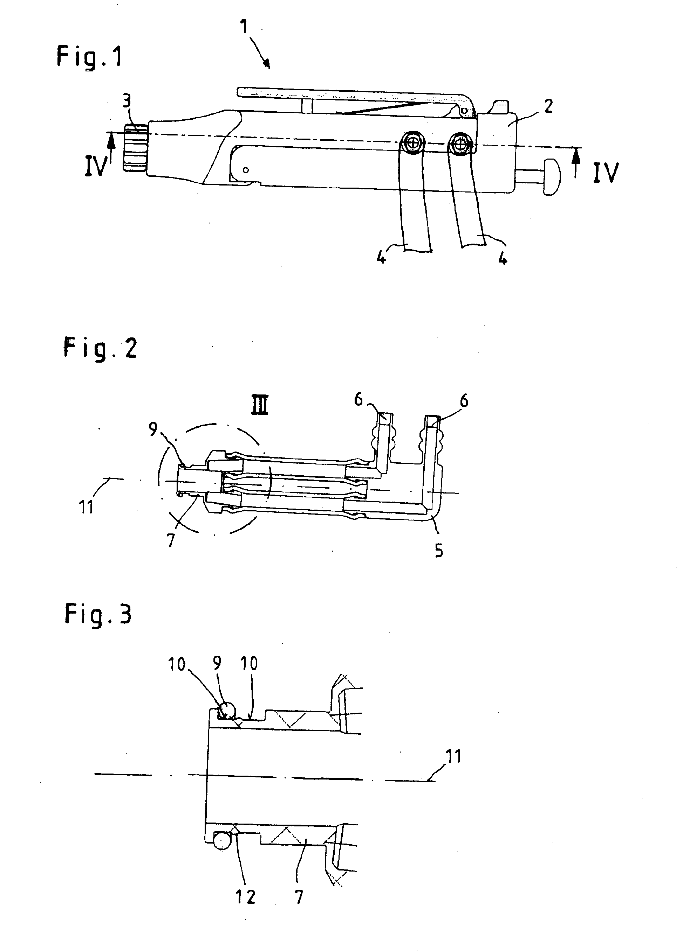

Die Abbildung Fig. 1 zeigt einen Handgriff 1 für ein nicht weiter dargestelltes medizinisches Instrument. Der Handgriff 1 weist ein Griffgehäuse 2 auf, an dessen distalem Ende beispielsweise eine Schraubkupplung 3 angeordnet ist, um den Handgriff 1 mit dem Schaft eines medizinischen Instruments zu verbinden.

Bei dem dargestellten Handgriff 1 handelt es sich um einen Saug-Spül-Handgriff 1, wie er beispielsweise in der endoskopischen Chirurgie Verwendung findet. Saug-Spül-Handgriffe 1 dienen dazu, dem Operationsgebiet des medizinischen Instruments über einen hohlen Schaft Spülflüssigkeit zuzuführen und Flüssigkeit aus dem Operationsgebiet abzusaugen. Die ausgehend von einer nicht dargestellten externen Spülflüssigkeitsquelle über den Handgriff 1 hin zum distalen Ende des medizinischen Instruments geführte Spülflüssigkeit dient während der Operation unter anderem dazu, eine Endoskopoptik zu reinigen, um dem Operateur einen stets ungetrübten Blick auf das Operationsgebiet zu gewährleisten. Über einen Saugkanal werden die Spülflüssigkeit und auch Blut aus dem Operationsgebiet abgesaugt, wobei der Saugkanal des medizinischen Instruments über den Handgriff 1 an eine nicht dargestellte externe Saugvorrichtung angeschlossen ist.

Zur Verbindung des Handgriffs 1 mit der externen Spülflüssigkeitsquelle und der externen Saugvorrichtung dienen Schlauchleitungen 4, die proximal handgriffseitig an einem im Griffgehäuse 2 angeordneten Anschlussadapter 5 festgelegt werden, wozu am Anschlussadapter 5 Schlauchanschlüsse 6 ausgebildet sind. Über einen distalseitig am Anschlussadapter 5 angeordneten Anschlussstutzen 7 wird der Anschlussadapter 5 fluiddicht in eine Stutzenaufnahme 8 des Griffgehäuses 2 eingesetzt.

Da der Anschlussadapter 5 nur schlecht oder gar nicht vollständig gereinigt werden kann, ist der Anschlussadapter 5 als auswechselbares Einwegteil ausgebildet.

Die fluiddichte Abdichtung im Bereich des Anschlussstutzens 7 des Anschlussadapters 5 und der Stutzenaufnahme 8 des Griffgehäuses 2 erfolgt über ein vorzugsweise als O-Ring ausgebildetes Dichtungselement 9, das in einer Ringnut 10 des Anschlussstutzens 7 angeordnet ist.

Die konstruktive Ausgestaltung des Anschlussadapters 5 sowie des mit dem Dichtungselement 9 versehenen Anschlussstutzens 7 ist insbesondere den Abbildungen Fig. 2 und 3 zu entnehmen.

Bei dem in Fig. 2 dargestellten Anschlussadapter 5 handelt es sich um einen Y-förmigen Anschlussadapters 5, der proximal seitlich zwei Schlauchanschlüsse 6 für Schlauchleitungen 4 und distalseitig einen Anschlussstutzen 7 zur Kontaktierung mit der Stutzenaufnahme 9 des Griffgehäuses 2 aufweist. Alternative Ausgestaltungen mit mehr oder weniger Schlauchanschlüssen 6 sowie mehreren Anschlussstutzen 7 sind ebenfalls konstruktiv möglich. Ebenso ist es möglich den oder die Anschlussstutzen 7 und oder den oder die Schlauchanschlüsse 6 räumlich anders am Anschlussadapter 5 anzuordnen.

Der Abbildung Fig. 3 ist der Aufbau der Dichtungsanordnung am Anschlussstutzen 7 zu entnehmen. Bei der dargestellten Ausführungsform sind in Axialrichtung des Anschlussstutzens 7 am Anschlussstutzen 7 zwei parallel zueinander angeordnete umlaufende Ringnuten 10 ausgebildet sind, wobei die beiden Ringnuten 10 in radialer Richtung gleich tief ausgebildet sind. Im in Fig. 2 und 3 dargestellten, noch nicht in das Griffgehäuse 2 eingesetzten Zustand des Anschlussadapters 5 ist das als O-Ring ausgebildete Dichtungselement 9 in der vom freien Ende des Anschlussstutzens 7 aus betrachtet ersten Ringnut 10 angeordnet.

Alternativ zu der parallelen Anordnung der beiden Ringnuten 10 zueinander ist es auch möglich, die Ringnuten schräg zueinander anzuordnen.

Die beiden parallel zueinander angeordneten Ringnuten 10 sind durch ein als radiale Erhebung ausgebildetes Stoppelement 12 voneinander getrennt.

Durch die Anordnung der zwei parallel zueinander angeordneten umlaufenden Ringnuten 10 und das in der distalseitig vordersten Ringnut 10 angeordnete Dichtungselement 9 wird beim Einschieben des Anschlussstutzens 7 in die Stutzenaufnahme 8 die auftretende Gleitreibung zwischen der Oberfläche des Dichtungselements 9 und der Innenseite der Stutzenaufnahme 8 dazu verwendet, das Dichtungselement 9 von der ersten Ringnut 10 in die zweite Ringnut 10 zu verschieben. Auf diese Weise wird der Widerstand beim Einsetzen des Anschlussadapters 5 in das Griffgehäuse 2 verringert und das Dichtungselement 9 trotzdem in einer genau definierten fluiddicht abdichtenden Position gehalten.

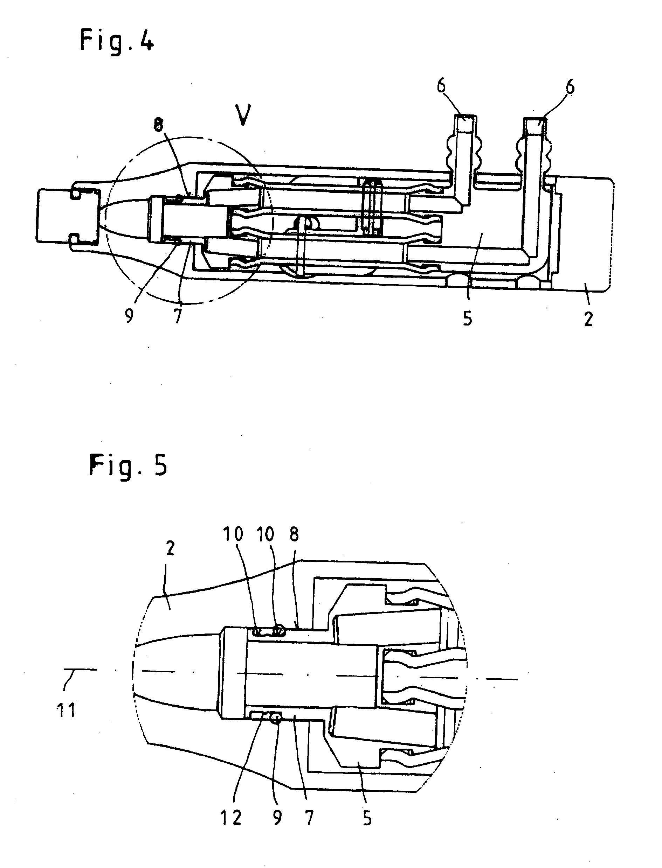

Da das Stoppelement 12 in Relation zum Dichtungselement 9 nur sehr klein, das heißt in axialer Richtung schmal und radial flach, ausgebildet ist, ist die Kraft, die benötigt wird, um das Dichtungselement 9 über das Stoppelement 12 zu schieben relativ gering. Durch die Bewegung des Einschiebens des Anschlussstutzens 7 in die Stutzenaufnahme 8 wird das Dichtungselement 9 gegen das Stoppelement 12 gedrückt und dreht sich um seinen halben Umfang. Um wieder in den Normalzustand zu gelangen, "springt" das Dichtungselement 9 quasi in die zweite Ringnut 10, so dass das Dichtungselement 9 bei vollständig in das Griffgehäuse 2 eingesetztem Anschlussadapter 5 nach einer vollen Umdrehung fest und gut abdichtend in der zweiten Ringnut 10 sitzt, wie dies den Abbildungen Fig. 4 und 5 zu entnehmen ist.

Wie weiterhin aus den Abbildungen Fig. 2 bis 5 ersichtlich, weist der Anschlussstutzen 7 im Bereich der zwei Ringnuten 10 einen gleichbleibenden Außendurchmesser auf, um eine gleichbleibende Kraft zum Überführen des Dichtungselements 9 von der einen Ringnut 10 in die andere Ringnut 10 zu gewährleisten.

Um eine definierte Lage des Dichtungselements 9 in der jeweiligen Ringnut 10 sicherzustellen, sind beide Ringnuten 10 gleich breit und in radialer Richtung gleich tief ausgebildet.

Bei der Demontage des Anschlussadapters 5 aus dem Griffgehäuse 2 wird das Dichtungselement 9 beim Herausziehen des Anschlussstutzens 7 aus der Stutzenaufnahme 8 durch die Bewegung des Herausziehens des Anschlussstutzens 7 aus der Stutzenaufnahme 8 gegen das Stoppelement 12 gedrückt und dreht sich um seinen halben Umfang. Um wieder in den Normalzustand zu gelangen, "springt" das Dichtungselement 9 quasi zurück in die erste Ringnut 10, so dass die Kraft beim Einsetzen des Anschlussadapters 5 genau so groß ist wie die beim Herausziehen des Anschlussadapters 5.

Alternativ zu der dargestellten Ausführungsform des Anschlussstutzens 7 mit zwei umlaufenden Ringnuten 10 und einem Dichtungselement 9 sind auch Ausgestaltungen mit mehr Ringnuten 10 und mehr Dichtungselementen 9 möglich.

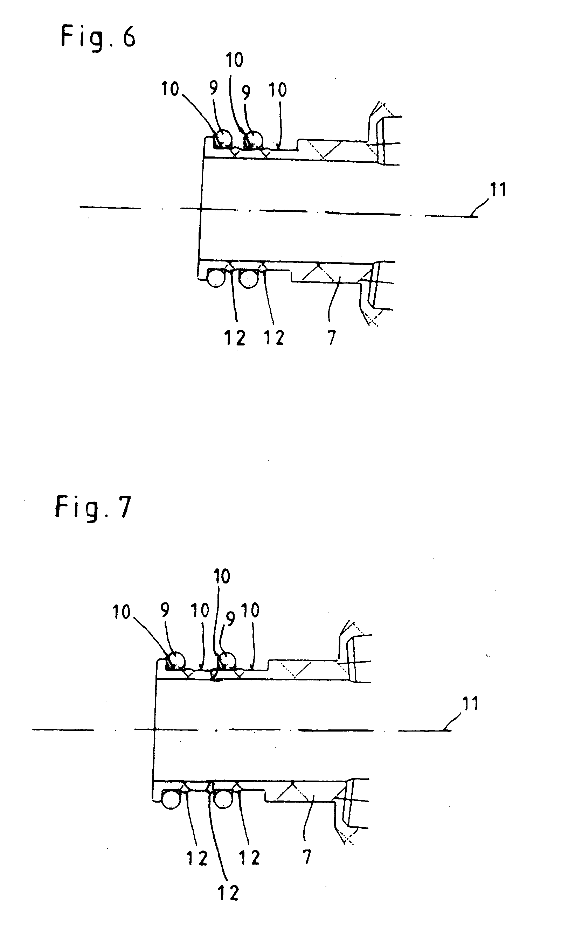

Nachfolgend werden anhand der Abbildungen Fig. 6 und 7 exemplarisch zwei alternative Ausgestaltungsformen zur Ausbildung der Anschlussstutzen 7 erläutert:

### Alternative 1 gemäß Fig. 6:

Ein Anschlussstutzen 7 mit drei parallelen Ringnuten 10 und zwei Dichtungselementen 9 die so angeordnet sind, dass die Dichtungselemente 9 bei der Montage des Anschlussadapters 5 von der ersten und zweiten Ringnut 10 in die zweite und dritte Ringnut 10 überführt werden.

### Alternative 2 gemäß Fig. 7:

Ein Anschlussstutzen 7 mit vier parallelen Ringnuten 10 und zwei Dichtungselementen 9 die so angeordnet sind, dass die Dichtungselemente 9 bei der Montage des Anschlussadapters 5 von der ersten und dritten Ringnut 10 in die zweite und vierte Ringnut 10 überführt werden.

Die voranstehend beschriebene Ausgestaltung der Anschlussstutzen 7 hat den Vorteil, dass die Rollreibung, die beim Einsetzen des Anschlussstutzens 7 des Anschlussadapters 5 in die Stutzenaufnahme 8 des Griffgehäuses 2 entsteht und das Dichtungselement 9 von der vorderen Ringnut 10 in die hintere Ringnut 10 überführt, das Einsetzen des Anschlussadapters 5 in das Griffgehäuse 2 erleichtert.

Durch diese Rollbewegung des Dichtungselements 9 wird das Auftreten der aus dem Stand der Technik bekannten hohen Gleitreibung verhindert, die bis zum Abscheren des O-Rings und somit zu einem undichten Instrument führen kann. Darüber hinaus wird das Toleranzspiel zwischen Dichtungselement 9, Anschlussstutzen 7 und Stutzenaufnahme 8 durch die Rollbewegung des Dichtungselements 9 ausgeglichen.

### Bezugszeichenliste

- 1: Handgriff
- 2: Griffgehäuse
- 3: Schraubkupplung
- 4: Schlauchleitung
- 5: Anschlussadapter
- 6: Schlauchanschluss
- 7: Anschlussstutzen
- 8: Stutzenaufnahme
- 9: Dichtungselement / O-Ring
- 10: Ringnut
- 11: Längsachse
- 12: Stoppelement

## Patentansprüche

1. Handgriff für ein medizinisches Instrument mit einem Griffgehäuse (2) und einem in das Griffgehäuse (2) einsetzbaren Anschlussadapter (5) für Schlauch- und/oder Rohranschlüsse, wobei der Anschlussadapter (5) über mindestens einen Anschlussstutzen (7) fluiddicht in eine entsprechende Stutzenaufnahme (8) des Griffgehäuses (2) einsetzbar ist und am mindestens einen Anschlussstutzen (7) eine umlaufende Ringnut (10) zur Aufnahme eines Dichtungselements (9) angeordnet ist, wobei in Axialrichtung des mindestens einen Anschlussstutzens (7) am Anschlussstutzen (7) mindestens zwei hintereinander angeordnete umlaufende Ringnuten (10) ausgebildet sind, wobei der Anschlussstutzen (7) im Bereich der mindestens zwei Ringnuten (10) einen gleichbleibenden Außendurchmesser aufweist, und wobei zumindest in der vom freien Ende des Anschlussstutzens (7) aus betrachtet ersten Ringnut (10) ein Dichtungselement (9), insbesondere ein O-Ring, angeordnet ist,
**dadurch gekennzeichnet,**
**dass** die zweite Ringnut (10) in Axialrichtung des Anschlussstutzens (7) nach proximal direkt an die erste Ringnut (10) anschließt und dass das mindestens eine Dichtungselement (9) beim Einsetzen des Anschlussstutzens (7) des Anschlussadapters (5) in die Stutzenaufnahme (8) des Griffgehäuses (2) durch. Gleitreibung aus seiner Lage in der ersten Ringnut (10) in die zweite Ringnut (10) überführbar ist.

2. Handgriff nach Anspruch 1, **dadurch gekennzeichnet, dass** die hintereinander angeordneten Ringnuten (10) durch jeweils ein Stoppelement (12) voneinander getrennt sind.

3. Handgriff nach Anspruch 2, **dadurch gekennzeichnet, dass** die Stoppelemente (12) als radiale Erhebungen ausgebildet sind.

4. Handgriff nach einem der Ansprüche 1 bis 3, **dadurch gekennzeichnet, dass** die hintereinander angeordneten Ringnuten (10) parallel zueinander angeordnet sind.

5. Handgriff nach einem der Ansprüche 1 bis 4, **dadurch gekennzeichnet, dass** die Ringnuten (10) gleich breit ausgebildet sind.

6. Handgriff nach einem der Ansprüche 1 bis 5, **dadurch gekennzeichnet, dass** die Ringnuten (10) in radialer Richtung gleich tief ausgebildet sind.

7. Handgriff nach einem der Ansprüche 1 bis 6, **dadurch gekennzeichnet, dass** am Anschlussstutzen (7) zwei in Axialrichtung parallel zueinander angeordnete umlaufende Ringnuten (10) ausgebildet sind.

8. Handgriff nach einem der Ansprüche 1 bis 7, **dadurch gekennzeichnet, dass** der Anschlussadapter (5) auswechselbar im Griffgehäuse (2) festlegbar ist.

9. Handgriff nach Anspruch 1, **dadurch gekennzeichnet, dass** das mindestens eine Dichtungselement (9) beim Herausziehen des Anschlussstutzens (7) des Anschlussadapters (5) aus der Stutzenaufnahme (8) des Griffgehäuses (2) wieder zurück in die ursprüngliche Ringnut (10) überführbar ist.

## Claims

1. A handle for a medical instrument with a grip housing (2) and a connecting adapter (5), which is insertable into the grip housing (2), for hose and/or pipe connections, wherein the connecting adapter (5) is insertable in a fluid-tight manner into a corresponding nozzle receptacle (8) of the grip housing (2) via at least one connection nozzle (7), and on the at least one connection nozzle (7), a circumferential annular groove (10) is arranged for receiving a sealing element (9), wherein, in the axial direction of the at least one connection nozzle (7), on the connection nozzle (7) at least two circumferential annular grooves (10) are formed, arranged one after the other, wherein the connection nozzle (7) in the area of the at least two annular grooves (10) has a constant outer diameter, and wherein, at least in the first annular groove (10), viewed from the free end of the connection nozzle (7), a sealing element (9), in particular an O ring, is arranged,
**characterized in that**
the second annular groove (10), in the axial direction of the connection nozzle (7), abuts proximally directly against the first annular groove (10) and **in that** the at least one sealing element (9), at the time of the insertion of the connection nozzle (7) of the connecting adapter (5) into the nozzle receptacle (8) of the grip housing (2) can be transferred by sliding friction from its position in the first annular groove (10) into the second annular groove (10).

2. A handle according to Claim 1, **characterized in that** the annular grooves (10), which are arranged one after the other, are separated from one another in each case by a stop element (12).

3. A handle according to Claim 2, **characterized in that** the stop elements (12) are formed as radial elevations.

4. A handle according to one of Claims 1 to 3, **characterized in that** the annular grooves (10), which are arranged one after another, are arranged parallel to one another.

5. A handle according to one of Claims 1 to 4, **characterized in that** the annular grooves (10) are formed so that they have the same width.

6. A handle according to one of Claims 1 to 5, **characterized in that** the annular grooves (10) are formed so that they have the same depth in the radial direction.

7. A handle according to one of Claims 1 to 6, **characterized in that**, on the connection nozzle (7), two circumferential annular grooves (10) are formed, which are arranged parallel to each other in the axial direction.

8. A handle according to one of Claims 1 to 7, **characterized in that** the connecting adapter (5) can be immobilized in a removable manner in the grip housing (2).

9. A handle according to Claim 1, **characterized in that**, at the time when the connection nozzle (7) of the connecting adapter (5) is pulled out of the nozzle receptacle (8) of the grip housing (2), the at least one sealing element (9) can be transferred back into the original annular groove (10).

## Revendications

1. Poignée pour un instrument médical comprenant un boitier de poignée (2) et un adaptateur de raccordement (5), qui peut être inséré dans le boitier de poignée (2) et qui est destiné à des raccords de branchement de tuyaux souples et/ou de tubulures, poignée dans laquelle l'adaptateur de raccordement (5) peut être inséré de manière étanche aux fluides, par l'intermédiaire d'au moins un embout de raccordement (7), dans un logement de réception d'embout (8) du boitier de poignée (2), et une rainure annulaire périphérique (10), destinée à recevoir un élément d'étanchéité (9), est agencée sur ledit au moins un embout de raccordement (7),
dans laquelle sur l'embout de raccordement (7) sont agencées, l'une à la suite de l'autre dans la direction axiale dudit au moins un embout de raccordement (7), au moins deux rainures annulaires périphériques (10),
dans laquelle l'embout de raccordement (7) présente, dans la zone desdites au moins deux rainures annulaires (10), un diamètre extérieur restant identique,
et dans laquelle un élément d'étanchéité (9), notamment un joint torique, est agencé au moins dans la première rainure annulaire (10) vu à partir de l'extrémité libre de l'embout de raccordement (7),
**caractérisée en ce que** la deuxième rainure annuaire (10) se raccorde directement à la première rainure annulaire (10) vers le côté proximal dans la direction axiale de l'embout de raccordement (7), et **en ce que** ledit au moins un élément d'étanchéité (9), lors de l'insertion de l'embout de raccordement (7) de l'adaptateur de raccordement (5) dans le logement de réception d'embout (8) du boitier de poignée (2), peut être transféré par friction de glissement, de sa position dans la première rainure annulaire (10), à la deuxième rainure annulaire (10).

2. Poignée selon la revendication 1, **caractérisée en ce que** les rainures annulaires (10) agencées les unes à la suite des autres sont séparées respectivement par un élément d'arrêt (12).

3. Poignée selon la revendication 2, **caractérisée en ce que** les éléments d'arrêt (12) sont réalisés sous la forme de saillies radiales.

4. Poignée selon l'une des revendications 1 à 3, **caractérisée en ce que** les rainures annulaires (10) agencées les unes à la suite des autres sont disposées parallèlement les unes aux autres.

5. Poignée selon l'une des revendications 1 à 4, **caractérisée en ce que** les rainures annulaires (10) sont réalisées de manière à présenter une largeur identique.

6. Poignée selon l'une des revendications 1 à 5, **caractérisée en ce que** les rainures annulaires (10) sont réalisées de manière à présenter la même profondeur dans la direction radiale.

7. Poignée selon l'une des revendications 1 à 6, **caractérisée en ce que** sur l'embout de raccordement (7) sont formées deux rainures annulaires périphériques (10) agencées parallèlement l'une à l'autre dans la direction axiale.

8. Poignée selon l'une des revendications 1 à 7, **caractérisée en ce que** l'adaptateur de raccordement (5) peut être fixé de manière interchangeable dans le boitier de poignée (2).

9. Poignée selon la revendication 1, **caractérisée en ce que** ledit au moins un élément d'étanchéité (9), lors du retrait de l'embout de raccordement (7) de l'adaptateur de raccordement (5) hors du logement de réception d'embout (8) du boitier de poignée (2), peut à nouveau être transféré dans la rainure annulaire (10) initiale.
